(19) Europäisches Patentamt / European Patent Office / Office européen des brevets

(11) **EP 4 031 068 B1**

(12) **EUROPÄISCHE PATENTSCHRIFT**

(45) Veröffentlichungstag und Bekanntmachung des Hinweises auf die Patenterteilung:
**17.04.2024 Patentblatt 2024/16**

(21) Anmeldenummer: **20764061.6**

(22) Anmeldetag: **25.08.2020**

(51) Internationale Patentklassifikation (IPC):
*A61F 2/14* (2006.01)   *G02B 27/00* (2006.01)
*G02B 27/01* (2006.01)

(52) Gemeinsame Patentklassifikation (CPC):
**A61F 2/14; G02B 27/0093; G02B 27/0172;**
G02B 2027/0138; G02B 2027/014;
G02B 2027/0178; G02B 2027/0187

(86) Internationale Anmeldenummer:
**PCT/EP2020/073744**

(87) Internationale Veröffentlichungsnummer:
**WO 2021/052725 (25.03.2021 Gazette 2021/12)**

(54) **MONITORSYSTEM FÜR EIN MENSCHLICHES ODER TIERISCHES AUGE**

MONITOR SYSTEM FOR A HUMAN OR ANIMAL EYE

SYSTÈME DE DISPOSITIF DE SURVEILLANCE POUR UN OEIL HUMAIN OU ANIMAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.09.2019 DE 102019124888**

(43) Veröffentlichungstag der Anmeldung:
**27.07.2022 Patentblatt 2022/30**

(73) Patentinhaber: **Deutsches Zentrum für Luft- und Raumfahrt e.V.**
**51147 Köln (DE)**

(72) Erfinder: **LORBEER, Raoul-Amadeus**
**71106 Magstadt (DE)**

(74) Vertreter: **RPK Patentanwälte**
**Reinhardt und Kaufmann**
**Partnerschaft mbB**
**Gaisburgstraße 21**
**70182 Stuttgart (DE)**

(56) Entgegenhaltungen:
WO-A1-2015/081298    WO-A2-2006/015315
US-A- 5 653 751      US-A1- 2009 189 830
US-A1- 2018 071 146

**Beschreibung**

**Stand der Technik**

Die Erfindung betrifft ein Monitorsystem für ein menschliches oder tierisches Auge.

Bei teilweisem oder vollständigem Verlust des Sehvermögens, etwa durch Zerstörung der Photorezeptorschicht in der Retina (auch Netzhaut genannt) des menschlichen Auges, werden häufig Retina-Implantate eingesetzt. Ein Retina-Implantat ist ein Array von Elektroden, die ähnlich einem Cochlea-Implantat in direkten Kontakt mit sekundären Nervenzellen gebracht werden. Hierdurch können bei Verlust der primären Sinneszellen die darauffolgenden Nervenzellen stimuliert und so ein Seheindruck erzeugt werden.

Retina-Implantate stimulieren die Nervenzellen direkt elektrisch. Dies ist typischerweise mit einer geringen räumlichen Auflösung verbunden, da die elektrischen Ströme verhältnismäßig weit in das Nervengewebe eindringen. Zudem muss das Implantat in direktem Kontakt zu der Retina stehen, damit sich dieser Effekt nicht weiter verstärkt. Retina-Implantate nutzen Elektroden zur Stimulation der sekundären Nervenzellen. Dies geschieht primär mit der Absicht, den Seheindruck wieder herzustellen und nicht die volle Sehkraft zu regenerieren. Die Methode erlaubt bisher lediglich rudimentäre Sinneswahrnehmungen zu generieren.

In der DE 10 2005 032 989 A1 ist beispielsweise eine Sehprothese mit einem Stimulations-System zur Implantation in einem menschlichen Auge beschrieben, welches ein Elektrodenarray zur Kontaktierung und Stimulation von lebendem Gewebe oder Nerven im visuellen System des Auges umfasst, das mittels einer elektrischen Schaltung Stimulations-Impulse erzeugt. Dabei umfasst das Stimulations-System zumindest ein intraokulares Implantat und zumindest ein extraokulares Implantat, welches das intraokulare Implantat mit Energie versorgt.

Erkrankungen des Auges, welche die Sehkraft stark reduzieren, jedoch nicht die Zerstörung der Photorezeptorschicht in der Retina zur Ursache haben und bei denen ein Retina-Implantat deshalb nicht eingesetzt wird, stellen beispielsweise eine Veränderung der Hornhaut, der Augenlinse, der Augenvorkammer oder aber auch des Glaskörpers dar. Ein weiteres aus medizinischer Sicht bisher ungelöstes Problem stellt beispielsweise die Altersweitsichtigkeit dar, welche typischerweise die meisten Menschen jenseits eines Lebensalters von vierzig Jahren betrifft. Weitere Behandlungsfelder umfassen Hornhautverkrümmung, leichte Formen des Sehfeldverlustes, Trübungen von Glaskörper, Linse oder Augenvorderkammer, weiter Fehlsichtigkeit generell, speziell schwierig korrigierbare Fehlsichtigkeit mit hohen Dioptrienzahlen.

Die US 2018/071146 A1 offenbart ein Monitorsystem für ein menschliches oder tierisches Auge, mit einem in das Auge implantierbaren Monitor, welcher mit einem Leistungsempfänger und einem Datenempfänger verbunden ist. Eine Kamera ist zur Aufnahme von Bilddaten vorhanden, welche mit einer ersten Übertragungseinheit zur Aufbereitung und zur drahtlosen Übertragung zumindest der Bilddaten über einen ersten Übertragungskanal an den Datenempfänger verbunden ist. Der Datenempfänger ist zum Empfang der Bilddaten sowie zum Weiterleiten an den Monitor ausgebildet. Der Monitor ist zur Darstellung von mit der Kamera aufgenommenen Bilddaten ausgebildet.

Die WO 2015/081298 A1 offenbart ein implantierbares Monitorsystem, das eine Linse als Optik aufweist, die natürliches Sehen erlaubt. Dabei wird Bildinformation auf die Oberfläche der Linse projiziert.

**Offenbarung der Erfindung**

Die Aufgabe der Erfindung ist es, ein Monitorsystem für ein menschliches oder tierisches Auge zu schaffen, welches bei stark reduzierter Sehkraft des Auges eine Verbesserung des Sehens ermöglicht.

Die Aufgabe wird durch die Merkmale des unabhängigen Anspruchs gelöst. Günstige Ausgestaltungen und Vorteile der Erfindung ergeben sich aus den weiteren Ansprüchen, der Beschreibung und der Zeichnung.

Nach einem Aspekt der Erfindung wird ein Monitorsystem für ein menschliches oder tierisches Auge vorgeschlagen, wenigstens umfassend einen in das Auge implantierbaren Monitor, welcher mit einem Leistungsempfänger und einem Datenempfänger verbunden ist, und eine Kamera zur Aufnahme von Bilddaten, welche mit einer ersten Übertragungseinheit zur Aufbereitung, insbesondere zur Komprimierung, und zur drahtlosen Übertragung zumindest der Bilddaten über einen ersten Übertragungskanal an den Datenempfänger verbunden ist. Der Datenempfänger ist zum Empfang und Decodieren der Bilddaten sowie zum Weiterleiten an den Monitor ausgebildet. Der Monitor ist dabei zur Darstellung von mit der Kamera aufgenommenen Bilddaten ausgebildet.

Der Monitor ist dazu vorgesehen, an Stelle der Linse im Linsensack des Auges anordenbar zu sein, insbesondere wobei auf einer Seite des Monitors, die einer Außenwelt zugewandt ist, der Leistungsempfänger und/oder der Datenempfänger angeordnet ist. Auf einer Seite des Monitors, die einer Innenseite des Auges zugewandt ist, ist eine Projektionseinrichtung vorgesehen, wodurch die Bilddaten auf die Netzhaut projizierbar sind. Der Monitor weist eine Öffnung in Form und Wirkung einer Pupille auf, wodurch Licht von einer Außenseite des Auges auf die Netzhaut fällt.

Dabei kann auf einer Seite des Monitors, die einer Innenseite des Auges zugewandt ist, eine Projektionseinrichtung vorgesehen sein, wodurch die Bilddaten auf die Netzhaut projizierbar sind.

Bei Fällen in denen der Glaskörper des Auges nicht ursächlich an der Sehverschlechterung beteiligt ist, kann eine Projektion genutzt werden um die Bildinformationen wieder auf die Netzhaut abzubilden. Hierbei kann der implantierte Monitor die Augenlinse ersetzen. Auf der der Außenwelt zugewandten Seite befindet sich der optische Leistungsempfänger für die Energieversorgung und der optische Datenempfänger für den Datenstrom. Auf der Innenseite in Richtung der Netzhaut ist ein Monitor als kompakte Projektionseinrichtung vorgesehen, der ein Bild auf die Netzhaut projiziert.

Das Monitorsystem kann zudem so ausgelegt sein, dass sich ein Loch in der Mitte befindet, welches wie eine Pupille wirkt und so bei hellen Umgebungsbedingungen auf natürliche Weise weiterhin das Sehen ermöglicht.

Das erfindungsgemäße Monitorsystem erlaubt vorteilhaft, Erkrankungen des Auges, welche die Sehkraft stark reduzieren, weil beispielsweise eine Veränderung der Hornhaut, der Augenlinse, der Augenvorkammer oder aber auch des Glaskörpers vorliegt, prothetisch zu umgehen und ein verbessertes Sehen zu ermöglichen.

Optional kann die Übertragungseinheit nicht nur zur drahtlosen Übertragung der Bilddaten, sondern auch zur drahtlosen Übertragung der Versorgungsleistung für Datenempfänger und/oder Monitor dienen. Diese Versorgungsleistung kann mittels optischen Signalen, z.B. Infrarotsignalen, per Funk, induktiv oder dergleichen, übertragen werden.

Die Sehkraft eines Auges kann durch eine fehlerhafte Abbildung auf die Netzhaut stark eingeschränkt sein. Derartige Erkrankungen können häufig nicht mit einer Brille korrigiert werden. Mit dem erfindungsgemäßen Monitorsystem ist es möglich, Bildinformation mit einer außerhalb des Auges angeordneten Kamera aufzunehmen und auf die Netzhaut abzubilden. Hierbei wird von der parallelen Bildübertragung, ein ganzes Bild auf einmal zu übertragen, auf eine serielle Bildübertragung, beispielsweise eine Punkt für Punkt-Übertragung, übergegangen. Das Bild wird extern mit der Kamera aufgenommen, komprimiert und in einen digitalen seriellen Datenstrom konvertiert. Wenn die Datenübertragung über die erste Übertragungseinheit auf optischem Weg erfolgt, kann der Datenstrom dann durch eine noch teilweise transparente Augenvorderkammer übertragen werden. Ein elektromagnetischer Datenstrom kann auch durch eine nicht mehr transparente Augenvorkammer übertragen werden.

Im Auge ist ein Monitor mit einem Leistungsempfänger und einem Datenempfänger als Implantat eingesetzt, welcher sich beispielsweise mit einem flexiblen Bildschirm an die Netzhaut anschmiegen kann und den digitalen, beispielsweise optischen, Datenstrom empfängt, dekodiert und auf die Netzhaut projiziert und so die Bildinformation wieder zur Verfügung stellt. Die Stromversorgung des implantierten Monitors kann beispielsweise auch durch eine Übertragung mittels Licht oder elektromagnetischer Strahlung gewährleistet werden. Alternativ kann eine Stromversorgung auch mittels einer langlebigen, vorzugsweise wiederaufladbaren Batterie erfolgen, welche wie bei einem Herzschrittmacher mitimplantiert werden kann.

Vorteilhaft kann das erfindungsgemäße Monitorsystem in der Ophthalmologie bei vielfältigen Sehstörungen, welche nicht den Verlust der primären Sinneszellen der Netzhaut, nämlich Stäbchen und Zäpfchen betreffen, eingesetzt werden.

Das Monitorsystem kann theoretisch auch zur über die medizinische Notwendigkeit hinausreichenden Verbesserung der Sehfähigkeit eingesetzt werden. Die Auflösungsgrenze der Netzhaut liegt bei ca. 300% Sehkraft.

Eine weitere mögliche Anwendung ist das Feld der Sinnens-Substitution. Hier können Sinneseindrücke technisch erfasst und über einen nicht von der Natur dafür vorgesehenen Sinn übertragen werden. Beispielsweise kann das Gehör über den Tastsinn am Rücken abgebildet werden. Da die Netzhaut die höchste Sinneszellendichte im Körper aufweist, können optional nicht-translatierende, also sich nicht mit der Augenbewegung verschiebende Datenströme, der Netzhaut aufgeprägt werden, um diese zum Ersatz von weiteren verlorenen Sinnen einzusetzen.

Gemäß einer vorteilhaften Ausgestaltung des Monitorsystems kann der Leistungsempfänger zum Empfang von elektromagnetischen Signalen, insbesondere optischen Signalen oder Funksignalen, und zur Umsetzung der elektromagnetischen Signale in elektrische Versorgungsleistung des Datenempfängers und des Monitors ausgebildet sein. Vorteilhaft ist eine dauerhafte Leistungsversorgung der implantierten Komponenten möglich.

Gemäß einer vorteilhaften Ausgestaltung des Monitorsystems kann eine zweite Übertragungseinheit zur drahtlosen Übertragung von Versorgungsleistung zur elektrischen Versorgung des Monitors und/oder Datenempfängers über einen zweiten Übertragungskanal an den Leistungsempfänger vorgesehen sein. Dabei kann der Leistungsempfänger vorteilhaft zum Empfang von elektromagnetischen Signalen und zur Umsetzung derselben zur elektrischen Versorgung des Datenempfängers und des Monitors ausgebildet sein. Eine Energieversorgung des Monitors sowie des Datenempfängers ist über einen drahtlosen Signalweg beispielsweise über das sogenannte Powerbeaming mit beispielsweise hochfrequenter elektromagnetischer Strahlung möglich. Alternativ ist jedoch auch optische Energieversorgung mittels Laser oder LEDs möglich. Dabei wird die elektrische Energie erst in optische Energie umgewandelt, in das Auge übertragen und dann in dem Leistungsempfänger empfangen und wieder in elektrische Energie zurückgewandelt. Auch über Induktoren ist eine drahtlose Energieversorgung ins Auge möglich.

Gemäß einer vorteilhaften Ausgestaltung des Monitorsystems kann die Kamera außerhalb des Auges angeordnet sein. Die Kamera kann zweckmäßig in der Nähe des Auges, im Bereich des Kopfs, angebracht sein, um den normalen Seheindruck beizubehalten. Die Kamera kann beispielsweise auf oder in einer Brille montiert sein. Auf diese Weise können die Bilddaten auch leicht durch die Linse in das Auge auf den Datenempfänger übertragen werden.

Gemäß einer vorteilhaften Ausgestaltung des Monitorsystems kann der Datenempfänger ausgebildet sein, digitale Bilddaten im Sichtfeld und/oder eine mit der Umgebung mitgeführte Darstellung in das Sichtfeld einzuspielen und/oder virtuelle Inhalte als Bilddaten auf den Monitor zu leiten.

Über die reine Reproduktion des Seheindruckes hinaus können auch zusätzliche Informationen in das Sichtfeld eingespielt werden. Diese Darstellungsform wird im Allgemeinen als Augmented Reality (AR) bezeichnet. Dies beinhaltet sowohl die fixe Darstellung von z.B. digitalen Bildinhalten im Sichtfeld als auch die mit der Umgebung mitgeführte Darstellung, so dass die Informationen sich scheinbar in einer bestimmten Richtung oder an einer bestimmten Position befinden. Als Bildinhalte sind alle Formate vorstellbar, die auch von Bildschirmen oder Fernsehern dargestellt werden können. In einer weiter gesteigerten Variante ist die Umgebung nicht mehr wahrzunehmen und lediglich virtuelle Inhalte werden dargestellt. Dies wird dann üblicherweise als Virtuelle Realität (VR) bezeichnet. Der Vorteil gegenüber üblichen AR und VR Systemen ist eine stets auf die Auflösung der Netzhaut angepasste Auflösung, so dass keine Abbildungsfehler durch zusätzliche optische Systeme hinzugefügt werden. Dies ist praktisch über das volle Sichtfeld möglich und reduziert gleichzeitig den Platzbedarf der Systeme, die vor das Auge gehalten werden müssen.

Gemäß einer vorteilhaften Ausgestaltung des Monitorsystems kann der erste Übertragungskanal von der ersten Übertragungseinheit durch die Linse zu dem Datenempfänger und/oder dem Leistungsempfänger ausgebildet sein. Weiter kann ein zweiter Übertragungskanal von der zweiten Übertragungseinheit durch die Linse zu dem Datenempfänger und/oder dem Leistungsempfänger ausgebildet sein. Wenn der erste und/oder zweite Übertragungskanal die Datenübertragung und/oder Leistungsübertragung auf optischem Weg realisiert, ist es zweckmäßig, wenn der Übertragungskanal als Lichtstrahl linear von der entsprechenden Übertragungseinheit durch die Linse zu der entsprechenden Empfangseinheit verläuft.

Gemäß einer vorteilhaften Ausgestaltung des Monitorsystems kann der erste Übertragungskanal als ein optischer Übertragungskanal, insbesondere im IR-Bereich, ausgebildet sein. Lichtstrahlen weisen den Vorteil auf, dass sie im Gegensatz zu elektromagnetischen Strahlen, wenig störanfällig gegenüber Beeinflussungen von außen sind. Ein Lichtstrahl im IR-Bereich ist vorteilhaft, da auf diese Weise eine Blendung im Auge ausgeschlossen ist.

Gemäß einer vorteilhaften Ausgestaltung des Monitorsystems kann der zweite Übertragungskanal als ein optischer Übertragungskanal, insbesondere im IR-Bereich, ausgebildet sein. Insbesondere kann die zweite Übertragungseinheit zur Umwandlung von elektrischer Leistung in optische Leistung und der Leistungsempfänger zur Umwandlung von optischer Leistung in elektrische Leistung ausgebildet sein.

Lichtstrahlen weisen den Vorteil auf, dass sie im Gegensatz zu anderen elektromagnetischen Strahlen wie Funk, wenig störanfällig gegenüber Beeinflussungen von außen sind. Ein Lichtstrahl im IR-Bereich ist vorteilhaft, da auf diese Weise eine Blendung im Auge ausgeschlossen ist. Elektrische Leistung kann auf optischem Weg günstig transportiert werden und im Leistungsempfänger auch wieder in elektrische Leistung umgewandelt werden, um den Datenempfänger und Monitor zu versorgen.

Vorteilhaft können die erste Übertragungseinheit und/oder die zweite Übertragungseinheit als Array von LEDs oder Laserdioden ausgebildet sein. Die beiden Übertragungseinheiten können baulich integriert vorgesehen sein. Dann können beispielsweise beide Übertragungskanäle in einen Kanal zusammengefasst sein.

Gemäß einer vorteilhaften Ausgestaltung des Monitorsystems kann der erste Übertragungskanal für eine serielle Übertragung von Bilddaten, insbesondere von komprimierten Bilddaten, ausgebildet sein. Eine serielle Übertragung von Bilddaten kann mit hoher Datenrate erfolgen, sodass trotzdem die Bilddaten eines aufgenommenen Bildes nahezu in Echtzeit übertragen werden können. Insbesondere, wenn die Bilddaten komprimiert sind, wofür sehr effiziente Algorithmen wie beispielsweise mpeg4 bzw. H.265 zur Verfügung stehen, kann die serielle Übertragung effizient durchgeführt werden.

Gemäß einer vorteilhaften Ausgestaltung des Monitorsystems kann der Monitor als ein flexibler Bildschirm ausgebildet sein, welcher dazu ausgebildet ist, auf einer Netzhaut des Auges direkt oder mit einem festen Abstand beabstandet vor der Netzhaut anordenbar zu sein.

Ein flexibler Bildschirm kann sich der natürlichen Wölbung des Augenhintergrundes mit der Netzhaut günstig anpassen und an die Netzhaut anschmiegen. Auf diese Weise können die Bilddaten von dem Monitor direkt auf die Netzhaut projiziert werden. Der Monitor kann dafür beispielsweise als µLED-Display, insbesondere als organischer LED-Display ausgebildet. Organische LED-Displays können als biegsame Folie hergestellt werden.

Der Monitor als flexibler Bildschirm kann auch mit einem geringen Abstand vor der Netzhaut angeordnet sein. Sollte ein direkter Kontakt mit der Netzhaut von Nachteil sein, kann eine Variation des Monitors eingesetzt werden, die lediglich mit einem Abstand von ca. 0,5 mm zur Netzhaut implantiert werden muss. So kann eine das ursprüngliche Kontaktproblem vermeidende Schicht zwischen Monitor und Netzhaut angebracht werden. Der Abstand von 0,5 mm ergibt sich aus der Tiefenschärfe einzelner Emitter von LEDs im sichtbaren Bereich des Lichts.

Für die Berechnung wird von einer optischen Auflösung auf der Netzhaut von 5 μm ausgegangen. Daher gilt bei einer Wellenlänge von 0,5 μm, dass die Tiefenschärfe in einem Medium mit Brechungsindex 1,34:

$$\Delta z = \frac{8{,}48 * 1{,}33 * (5\,\mu m)^2}{0{,}5\,\mu m} = 563{,}92\,\mu m$$

beträgt (nach Raoul-Amadeus Lorbeer, "Quantifizierung optischer Systeme für die optische Tomographie", Dissertation, Gottfried Wilhelm Leibniz Universität Hannover, 2012).

Gemäß einer vorteilhaften Ausgestaltung des Monitorsystems kann eine Bewegung des Auges mit einem System zur Verfolgung von Augenbewegungen verfolgbar sein. Weiter kann der Monitor mit Beschleunigungssensoren ausgebildet sein, welche mit der Kamera und/oder der ersten Übertragungseinheit so zusammenwirken, dass eine Bildstabilisierung erfolgt. Zur korrekten Darstellung kann das übertragene Bild zuvor an die Augenbewegung angeglichen werden.

So ist es vorteilhaft, die Augenbewegung mit einem System zur Verfolgung von Augenbewegungen, einem sogenannten Eye-Tracker, zu verfolgen und redundant hierzu Beschleunigungssensoren im Monitor unterzubringen, die bei der Bildstabilisierung unterstützende Informationen an die Kamera und/oder Übertragungseinheit zur Verfügung senden.

Gemäß einer vorteilhaften Ausgestaltung des Monitorsystems kann ein Kompressionsalgorithmus für die Bilddaten an eine typische Schärfenverteilung des Auges angepasst sein. Zudem kann der Kompressionsalgorithmus so an die typische Schärfenverteilung des Auges angepasst werden, so dass nicht unnötig detailreiche Bildinformationen an das äußere Gesichtsfeld gesendet werden.

Gemäß einer vorteilhaften Ausgestaltung des Monitorsystems können auf dem Monitor dargestellte Bilddaten so verzerrt werden, dass Bildelemente in ausgewählte Bereiche der Netzhaut projizierbar sind. Auf diese Weise kann eine Anpassung des Gesichtsfelds erfolgen. Durch die fixe Anordnung des Monitors/Projektors ist es möglich, Gesichtsfeldverluste teilweise zu kompensieren.

Das Bild kann in einigen Bereichen so verzerrt werden, dass Bildelemente, die sonst auf ausgefallene Bereiche der Netzhaut treffen, von den umgebenden Sinneszellen wahrgenommen und somit von der Person verarbeitet werden können.

Vorteilhaft kann der Monitor als semitransparenter Monitor ausgebildet sein. Um die verbliebene Sehkraft bei Nicht-Nutzung oder Ausfall des Monitorsystems zu erhalten ist es möglich, den Monitor semitransparent zu gestalten und den Datenempfänger und/oder Leistungsempfänger in großen Sehwinkeln zu installieren. Die Installation des Empfängers am Rand des Gesichtsfeldes ist auf Grund der Geometrie der Übertragungseinheiten, welche von der Person getragen werden, generell vorzuziehen.

Vorteilhaft kann der Monitor zum Leuchten und zum Empfangen von Licht ausgebildet sein. Insbesondere kann der Monitor als μLED-Display, insbesondere als organischer LED-Display ausgebildet sein. Bei einem μ-LED Display ist es möglich, die einzelnen Pixel sowohl zum Leuchten als auch zum Empfangen von Licht zu nutzen. Dies erlaubt es, Methoden der diffusen optischen Tomographie einzusetzen und dreidimensionale Aufnahmen der Netzhaut zu erhalten. So könnte die Netzhaut des Implantatträgers regelmäßig detailliert untersucht werden, beispielsweise um auf Verschlechterungen des Sehvermögens schneller reagieren zu können.

Gemäß einer vorteilhaften Ausgestaltung des Monitorsystems können der Leistungsempfänger und der Datenempfänger in einer Einheit integriert ausgebildet sein. Insbesondere können der Leistungsempfänger und der Datenempfänger in einer Einheit in den Monitor integriert ausgebildet sein. Dadurch lässt sich ein kompakter Monitor realisieren, welcher vorteilhaft für eine Implantation im Auge geeignet ist.

Gemäß einer vorteilhaften Ausgestaltung des Monitorsystems können die erste Übertragungseinheit und die zweite Übertragungseinheit in einer Einheit integriert ausgebildet sein. Insbesondere können die erste Übertragungseinheit und die zweite Übertragungseinheit in einer Einheit in die Kamera integriert ausgebildet sind. Dadurch lässt sich ein kompaktes Kamerasystem darstellen, welches mit geringem Gewicht und kleinem Bauraum beispielsweise in einer Brille unterzubringen ist.

Gemäß einer vorteilhaften Ausgestaltung des Monitorsystems können die Kamera, insbesondere ein Scharfstellen der Kamera über einen Ziliarkörper des Auges durch die Spannung der Ziliarmuskeln ansteuerbar sein. Weiter kann die Kamera, insbesondere ein Scharfstellen der Kamera über ein System zur Verfolgung von Augenbewegungen ansteuerbar sein. Auf diese Weise lässt sich mit dem Monitorsystem ein möglichst realistischer Seheindruck für den Implantatträger verwirklichen.

Mit dem Monitorsystem für ein menschliches oder tierisches Auge mit einem in das Auge implantierbaren Monitor, welcher mit einem Leistungsempfänger und einem Datenempfänger verbunden ist, können Bilddaten aufgenommen werden; Bilddaten an den Datenempfänger übermittelt werden; Bilddaten vom Datenempfänger an den Monitor weitergeleitet werden und der Monitor die Bilddaten im Auge darstellen.

Die Bilddaten können von einer Kamera außerhalb des Auges erfasst werden. Vorteilhaft kann ein Scharfstellen der Kamera über einen Ziliarkörper des Auges durch die Spannung der Ziliarmuskeln ansteuerbar sein.

Auf einer Seite des Monitors, die einer Innenseite des Auges zugewandt ist, ist eine Projektionseinrichtung vorgesehen, wodurch die Bilddaten auf die Netzhaut projizierbar sind. Der Monitor weist eine Öffnung in Form und Wirkung einer Pupille auf, wodurch Licht von einer Außenseite des Auges auf die Netzhaut fällt.

Dabei kann auf einer Seite des Monitors, die einer Innenseite des Auges zugewandt ist, eine Projektionseinrichtung vorgesehen sein, wodurch die Bilddaten auf die Netzhaut projizierbar sind.

Bei Fällen in denen der Glaskörper des Auges nicht ursächlich an der Sehverschlechterung beteiligt ist, kann eine Projektion genutzt werden um die Bildinformationen wieder auf die Netzhaut abzubilden. Hierbei kann der implantierte Monitor die Augenlinse ersetzen. Auf der der Außenwelt zugewandten Seite befindet sich der optische Leistungsempfänger für die Energieversorgung und der optische Datenempfänger für den Datenstrom. Auf der Innenseite in Richtung der Netzhaut ist ein Monitor als kompakte Projektionseinrichtung vorgesehen, der ein Bild auf die Netzhaut projiziert.

Das Monitorsystem kann zudem so ausgelegt sein, dass sich ein Loch in der Mitte befindet, welches wie eine Pupille wirkt und so bei hellen Umgebungsbedingungen auf natürliche Weise weiterhin das Sehen ermöglicht.

Die Bilddaten können von einer Kamera außerhalb des Auges erfasst werden. Vorteilhaft kann ein Scharfstellen der Kamera über einen Ziliarkörper des Auges durch die Spannung der Ziliarmuskeln ansteuerbar sein.

Weiter kann die Kamera, insbesondere ein Scharfstellen der Kamera über ein System zur Verfolgung von Augenbewegungen ansteuerbar sein. Auf diese Weise lässt sich mit dem Monitorsystem ein möglichst realistischer Seheindruck für den Implantatträger verwirklichen.

Es kann eine Versorgungsleistung des Datenempfängers und des Monitors drahtlos übertragen werden. Dies erlaubt eine einfache und dauerhafte Versorgung des Monitorsystems.

Die Übertragung von Bilddaten, insbesondere von komprimierten Bilddaten, kann seriell erfolgen. Eine serielle Übertragung von Bilddaten kann mit hoher Datenrate erfolgen, sodass trotzdem die Bilddaten eines aufgenommenen Bildes nahezu in Echtzeit übertragen werden können. Insbesondere, wenn die Bilddaten komprimiert sind, wofür sehr effiziente Algorithmen wie beispielsweise mpeg4 bzw. H.265 zur Verfügung stehen, kann die serielle Übertragung effizient durchgeführt werden.

Ein Kompressionsalgorithmus kann so an die typische Schärfenverteilung des Auges angepasst werden, dass eine Weitergabe von unnötig detailreichen Bildinformationen an das äußere Gesichtsfeld zumindest reduziert wird.

Es können auf dem Monitor dargestellte Bilddaten verzerrt werden, so dass Bildelemente in ausgewählte Bereiche der Netzhaut projiziert werden. Auf diese Weise kann eine Anpassung des Gesichtsfelds erfolgen. Durch die fixe Anordnung des Monitors/Projektors ist es möglich, Gesichtsfeldverluste teilweise zu kompensieren.

Das Bild kann in einigen Bereichen so verzerrt werden, dass Bildelemente, die sonst auf ausgefallene Bereiche der Netzhaut treffen, von den umgebenden Sinneszellen wahrgenommen und somit von der Person verarbeitet werden können.

Digitale Bilddaten im Sichtfeld und/oder eine mit der Umgebung mitgeführte Darstellung können in das Sichtfeld eingespielt werden und/oder es können virtuelle Inhalte als Bilddaten auf den Monitor geleitet werden.

Über die reine Reproduktion des Seheindruckes hinaus können auch zusätzliche Informationen in das Sichtfeld eingespielt werden. Diese Darstellungsform wird im Allgemeinen als Augmented Reality (AR) bezeichnet. Dies beinhaltet sowohl die fixe Darstellung von z.B. digitalen Bildinhalten im Sichtfeld als auch die mit der Umgebung mitgeführte Darstellung, so dass die Informationen sich scheinbar in einer bestimmten Richtung oder an einer bestimmten Position befinden. Als Bildinhalte sind alle Formate vorstellbar, die auch von Bildschirmen oder Fernsehern dargestellt werden können. In einer weiter gesteigerten Variante ist die Umgebung nicht mehr wahrzunehmen und lediglich virtuelle Inhalte werden dargestellt. Dies wird dann üblicherweise als Virtuelle Realität (VR) bezeichnet. Der Vorteil gegenüber üblichen AR und VR Systemen ist eine stets auf die Auflösung der Netzhaut angepasste Auflösung, so dass keine Abbildungsfehler durch zusätzliche optische Systeme hinzugefügt werden. Dies ist praktisch über das volle Sichtfeld möglich und reduziert gleichzeitig den Platzbedarf der Systeme, die vor das Auge gehalten werden müssen.

## Zeichnung

Weitere Vorteile ergeben sich aus der folgenden Zeichnungsbeschreibung. In den Figuren sind Ausführungsbeispiele der Erfindung dargestellt. Die Figuren, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

**Es zeigen beispielhaft:**

Fig. 1 einen Längsschnitt durch ein menschliches Auge mit einem Monitorsystem nach einem Ausführungsbeispiel der Erfindung;

Fig. 2 einen Längsschnitt durch ein menschliches Auge mit einem Monitorsystem nach einem weiterem Ausführungsbeispiel der Erfindung;

Fig. 3 ein Flussdiagramm eines vorteilhaften Verfahrens zum Betreiben eines Monitorsystems.

**Ausführungsformen der Erfindung**

In den Figuren sind gleichartige oder gleichwirkende Komponenten mit gleichen Bezugszeichen beziffert. Die Figuren zeigen lediglich Beispiele und sind nicht beschränkend zu verstehen.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

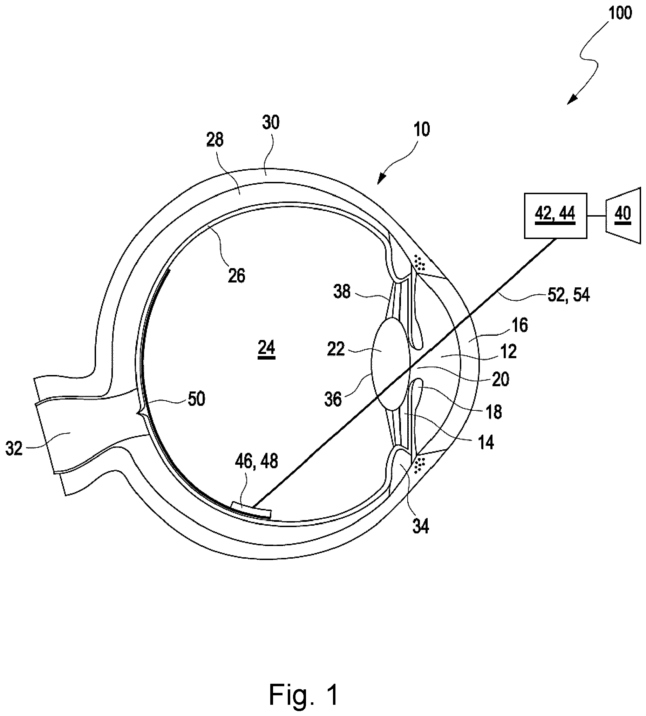

Figur 1 zeigt beispielhaft einen Längsschnitt durch ein menschliches Auge 10 mit einem Monitorsystem 100 zur Implantation in das Auge nach einem Ausführungsbeispiel der Erfindung.

Das Monitorsystem 100 umfasst den in das Auge 10 implantierbaren Monitor 50, welcher mit einem Leistungsempfänger 46 und einem Datenempfänger 48 verbunden ist. Der Monitor 50 liegt als flexible Folie, beispielsweise als ein μLED-Display, insbesondere als ein organischer OLED-Display, am Augenhintergrund auf der Netzhaut 26 auf. Die Netzhaut 26 ist wiederum als innerste Schicht auf der Aderhaut 28 und der darunterliegenden Lederhaut 30 ausgebildet. Sinneseindrücke der Netzhaut 26 werden über den Sehnerv 32 an das Gehirn weitergeleitet. Der Leistungsempfänger 46 und der Datenempfänger 48 sind als integrierte Einheit direkt an den Monitor 50 elektrisch und mechanisch angeschlossen.

Optional kann der Monitor 50 beabstandet zu der Netzhaut 26 angeordnet sein und beispielsweise der Krümmung der Netzhaut 26 angepasst sein (nicht dargestellt).

Der Monitor 50 kann semitransparent ausgebildet sein, um die verbliebene Sehkraft des Auges 10 bei Ausfall des Monitorsystems 100 oder bei Nicht-Nutzung desselben ausnutzen zu können.

Weiter umfasst das Monitorsystem 100 eine Kamera 40 zur Aufnahme von Bilddaten, welche mit einer ersten Übertragungseinheit 42 zur Aufbereitung, insbesondere zur Komprimierung und drahtlosen Übertragung der Bilddaten über einen ersten Übertragungskanal 52 an den Datenempfänger 48 verbunden ist. Die Kamera 40 mit der integrierten ersten Übertragungseinheit 42 für Bilddaten und der zweiten Übertragungseinheit 44 für elektrische Leistung, welche in einer Einheit integriert sind, ist außerhalb und vor dem Auge 10 angeordnet.

Der Datenempfänger 48 empfängt und dekodiert die Bilddaten und leitet sie an den Monitor 50 weiter. Der Monitor 50 stellt die mit der Kamera 40 aufgenommenen Bilddaten auf der Netzhaut 26 dar.

Die zweite Übertragungseinheit 44 ist zur drahtlosen Übertragung von elektrischer Leistung über einen zweiten Übertragungskanal 54 an den Leistungsempfänger 46 vorgesehen. Der Leistungsempfänger 46 empfängt die elektrische Leistung und stellt die elektrische Versorgung von Datenempfänger 48 und Monitor 50 sicher.

Der erste Übertragungskanal 52 verläuft von der ersten Übertragungseinheit 42 durch die Linse 22 zu dem Datenempfänger 48 und/oder dem Leistungsempfänger 46. Der zweite Übertragungskanal 54 verläuft von der zweiten Übertragungseinheit 44, durch die Linse 22 zu dem Datenempfänger 48 und/oder dem Leistungsempfänger 46. Die beiden Übertragungskanäle 52, 54 können als ein Übertragungskanal durch die transparente Hornhaut 16 in die vordere Augenkammer 12 eintreten, durch die Pupille 20 in die hintere Augenkammer 14 durchtreten, sowie dann durch die von den Zonulafasern 38 des Ziliarkörpers 34 gehaltenen Linse 22 und durch den Glaskörper 24 auf den Datenempfänger 48, bzw. Leistungsempfänger 46 treffen.

Sowohl der erste Übertragungskanal 52 als auch der zweite Übertragungskanal 54 können als ein optischer Übertragungskanal, insbesondere im IR-Bereich, ausgebildet sein. Die zweite Übertragungseinheit 44 wandelt dabei elektrische Leistung in optische Leistung und der Leistungsempfänger 46 wandelt wiederum optische Leistung in elektrische Leistung um.

Der erste Übertragungskanal 52 überträgt die Bilddaten, insbesondere von komprimierten Bilddaten, vorzugsweise seriell.

Der Monitor 50 kann zweckmäßig als ein flexibler Bildschirm ausgebildet sein, welcher auf der Netzhaut 26 des Auges 10 direkt oder mit einem festen Abstand beabstandet vor der Netzhaut 26 angeordnet ist.

Eine Bewegung des Auges 10 kann in einer weiteren Ausführungsform mit einem System zur Verfolgung von Augenbewegungen verfolgt werden. Weiter kann der Monitor 50 in einer nicht dargestellten Ausgestaltung mit Beschleunigungssensoren ausgebildet sein, welche mit der Kamera 50 und/oder der ersten Übertragungseinheit 42 so zusammenwirken, dass eine Bildstabilisierung erfolgt. Hierfür ist ein Rückkanal vom Implantat zur Außenwelt vorgesehen, der ebenso wie der erste und/oder zweite Übertragungskanal drahtlos vorgesehen ist und elektromagnetische Signale, z.B. optische Signale oder Funksignale oder induktive Signale übertragen kann. Zur korrekten Darstellung kann das übertragene Bild zuvor an die Augenbewegung angeglichen werden. Die Daten können bei der Bildstabilisierung unterstützende Informationen an die Kamera 50 und/oder die Übertragungseinheit 42 zur Verfügung senden. Ein Kompressionsalgorithmus für die Bilddaten kann dabei an eine typische Schärfenverteilung des Auges 10 angepasst sein.

Auf dem Monitor 50 dargestellte Bilddaten können so verzerrt werden, dass Bildelemente, welche auf etwaige ausgefallene Teile der Netzhaut 26 fallen würden, in ausgewählte Bereiche der Netzhaut 26 projiziert werden, insbesondere auf Bereiche, welche die ausgefallenen Teil der Netzhaut 26 umgeben. Dazu können der Leistungsempfänger 46 und/oder der Datenempfänger 48 am Rand eines Bildfeldes im Auge 10 angeordnet sein.

Um den Monitor 50 in weiterer günstiger, nicht dargestellter Ausgestaltung optional zu einer Art Retina-Tomographie nutzen zu können, ist es zweckmäßig, wenn der Monitor 50 zum Leuchten und zum Empfangen von Licht ausgebildet ist, insbesondere als organischer LED-Display.

So kann die Netzhaut 26 regelmäßig detailliert auf Veränderungen untersucht werden. Hierfür ist ein Rückkanal vom Implantat zur Außenwelt vorgesehen, der ebenso wie der erste und/oder zweite Übertragungskanal drahtlos vorgesehen ist und elektromagnetische Signale, z.B. optische Signale oder Funksignale oder induktive Signale übertragen kann.

Die Kamera 40, insbesondere ein Scharfstellen der Kamera 40 kann vorteilhaft über den Ziliarkörper 34 durch die Spannung der Ziliarmuskeln angesteuert werden, sodass ein natürlicher Seheindruck erhalten bleiben kann. Zusätzlich kann die Kamera 40, insbesondere ein Scharfstellen der Kamera 40 über ein System zur Verfolgung von Augenbewegungen angesteuert werden.

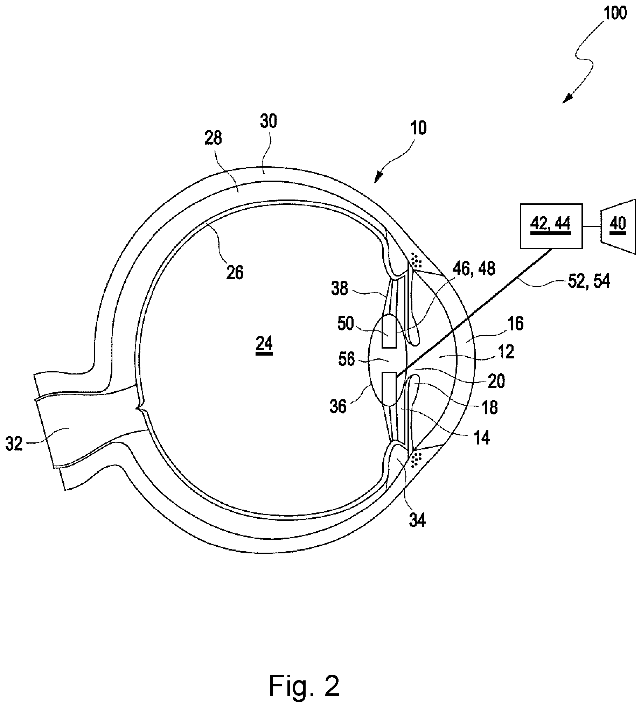

Figur 2 zeigt einen Längsschnitt durch ein menschliches Auge 10 mit einem Monitorsystem 100 nach einem weiteren Ausführungsbeispiel der Erfindung, bei dem der Monitor 50 die Linse ersetzt. Die übrige Ausführung des Monitorsystems 100 entspricht der in Figur 1 dargestellten Ausführungsform. Zur Vermeidung unnötiger Wiederholungen wird auf die vorangegangene Figurenbeschreibung verwiesen.

Bei der in Figur 2 dargestellten Ausführungsform ist der Monitor 50 an Stelle der Linse 22 im Linsensack 36 des Auges 10 angeordnet. Auf der Seite des Monitors 50, die einer Außenwelt zugewandt ist, ist der Leistungsempfänger 46 und/oder der Datenempfänger 48 angeordnet. Auf der Seite des Monitors 50, die einer Innenseite des Auges 10 zugewandt ist, ist eine Projektseinrichtung vorgesehen, wodurch die Bilddaten auf die Netzhaut 26 projizierbar sind.

Eine solche Ausführungsform kann eingesetzt werden, wenn der Glaskörper 24 nicht ursächlich an der Sehverschlechterung beteiligt ist und die Linse 22 völlig geschädigt ist, sodass sie entfernt werden kann. Dafür können die Bildinformationen von dem Projektionssystem des Monitors 50 auf die Netzhaut 26 abgebildet werden.

Zusätzlich weist der Monitor 50 eine Öffnung 56 in Form und Wirkung einer Pupille aufweist, wodurch Licht von einer Außenseite des Auges 10 auf die Netzhaut 26 fällt. Die Öffnung 56 befindet sich in der Mitte, und wirkt wie eine Pupille. Dadurch wird bei hellen Umgebungsbedingungen auf natürliche Weise weiterhin das Sehen ermöglicht.

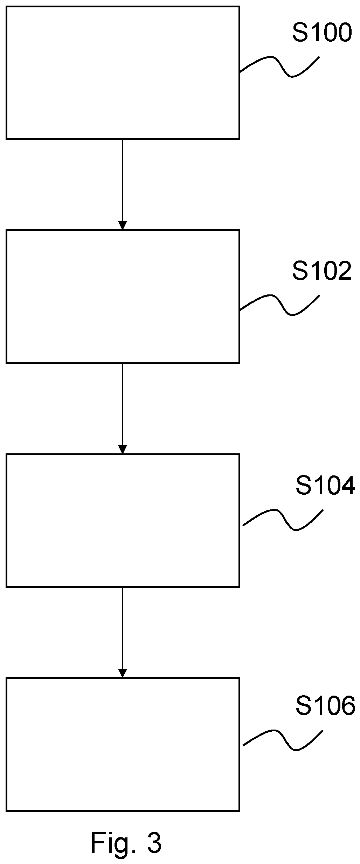

Figur 3 zeigt ein Flussdiagramm eines vorteilhaften Verfahrens zum Betreiben eines Monitorsystems 100 für ein menschliches oder tierisches Auge 10. Das Monitorsystem 100 kann beispielsweise ausgestaltet sein wie vorstehend beschrieben wurde und ein in das Auge 10 implantierbaren Monitor 50 aufweisen, welcher mit einem Leistungsempfänger 46 und einem Datenempfänger 48 verbunden ist.

In Schritt S100 werden Bilddaten aufgenommen. Die Bilddaten können von einer Kamera außerhalb des Auges aufgenommen werden.

In Schritt S102 werden Bilddaten an den Datenempfänger 48 übermittelt. Zur Übermittlung können die Bilddaten komprimiert werden. Die Übertragung von Bilddaten, insbesondere von komprimierten Bilddaten, erfolgt vorzugsweise seriell. Bilddaten können mit hoher Datenrate übermittelt werden, sodass trotzdem die Bilddaten eines aufgenommenen Bildes nahezu in Echtzeit übertragen werden können. Ein Kompressionsalgorithmus kann so an die typische Schärfenverteilung des Auges angepasst werden, dass eine Weitergabe von unnötig detailreichen Bildinformationen an das äußere Gesichtsfeld zumindest reduziert wird.

In Schritt S104 werden die Bilddaten vom Datenempfänger 48 an den Monitor 50 weitergeleitet.

In Schritt S106 stellt der Monitor 50 die Bilddaten im Auge 10 dar. Auf dem Monitor 50 dargestellte Bilddaten können so verzerrt werden, dass Bildelemente in ausgewählte Bereiche der Netzhaut 26 projiziert werden. So können Gesichtsfeldverluste teilweise kompensiert werden. Das Bild kann in einigen Bereichen so verzerrt werden, dass Bildelemente, die sonst auf ausgefallene Bereiche der Netzhaut treffen, von den umgebenden Sinneszellen wahrgenommen und somit von der Person verarbeitet werden können.

Eine Versorgung des Datenempfängers 48 und des Monitors 50 erfolgt vorzugsweise drahtlos.

| | |
|---|---|
| 10 | Auge |
| 12 | vordere Augenkammer |
| 14 | hintere Augenkammer |
| 16 | Hornhaut |
| 18 | Iris |
| 20 | Pupille |
| 22 | Linse |
| 24 | Glaskörper |
| 26 | Netzhaut |
| 28 | Aderhaut |
| 30 | Lederhaut |
| 32 | Sehnerv |
| 34 | Ziliarkörper |
| 36 | Linsensack |
| 38 | Zonulafaser |
| 40 | Kamera |
| 42 | Übertragungseinheit für Bilddaten |
| 44 | Übertragungseinheit für elektr. Leistung |
| 46 | Leistungsempfänger |
| 48 | Datenempfänger |
| 50 | Monitor |
| 52 | Übertragungskanal |
| 54 | Übertragungskanal |
| 56 | Öffnung |
| 100 | Monitorsystem |

S100    Bilddaten aufnehmen
S102    Bilddaten übermitteln
S104    Bilddaten weiterleiten
S106    Bilddaten darstellen

**Patentansprüche**

1.  Monitorsystem (100) für ein menschliches oder tierisches Auge (10), wenigstens umfassend

    einen in das Auge (10) implantierbaren Monitor (50), welcher mit einem Leistungsempfänger (46) und einem Datenempfänger (48) verbunden ist,
    eine Kamera (40) zur Aufnahme von Bilddaten, welche mit einer ersten Übertragungseinheit (42) zur Aufbereitung, insbesondere Komprimierung, und zur drahtlosen Übertragung zumindest der Bilddaten über einen ersten Übertragungskanal (52) an den Datenempfänger (48) verbunden ist,
    wobei der Datenempfänger (48) zum Empfang der Bilddaten sowie zum Weiterleiten an den Monitor (50) ausgebildet ist,
    wobei der Monitor (50) zur Darstellung von mit der Kamera (40) aufgenommenen Bilddaten ausgebildet ist,
    wobei der Monitor (50) dazu vorgesehen ist, an Stelle der Linse (22) im Linsensack (36) des Auges (10) anordenbar zu sein, insbesondere wobei auf einer Seite des Monitors (50), die einer Außenwelt zugewandt ist, der Leistungsempfänger (46) und/oder der Datenempfänger (48) angeordnet ist, und wobei auf einer Seite des Monitors (50), die einer Innenseite des Auges (10) zugewandt ist, eine Projektionseinrichtung vorgesehen ist, wodurch die Bilddaten auf die Netzhaut (26) projizierbar sind,
    **dadurch gekennzeichnet, dass**
    der Monitor (50) eine Öffnung (56) in Form und Wirkung einer Pupille aufweist, wodurch Licht von einer Außenseite des Auges (10) auf die Netzhaut (26) fällt.

2.  Monitorsystem nach Anspruch 1, **dadurch gekennzeichnet, dass** der Leistungsempfänger (46) zum Empfang von elektromagnetischen Signalen, insbesondere optischen Signalen oder Funksignalen, und zur Umsetzung der elektromagnetischen Signale in elektrische Versorgungsleistung des Datenempfängers (48) und des Monitors (50) ausgebildet ist.

3.  Monitorsystem nach Anspruch 1 oder 2, **dadurch gekennzeichnet, dass** eine zweite Übertragungseinheit (44) zur drahtlosen Übertragung von Versorgungsleistung zur elektrischen Versorgung des Datenempfängers (48) und/oder des Monitors (50) über einen zweiten Übertragungskanal (54) an den Leistungsempfänger (46) vorgesehen ist.

4.  Monitorsystem nach einem der vorhergehenden Ansprüche, **dadurch gekennzeichnet, dass** die Kamera (40) außerhalb des Auges (10) angeordnet ist.

5.  Monitorsystem nach einem der vorhergehenden Ansprüche, **dadurch gekennzeichnet, dass** der Datenempfänger (48) ausgebildet ist, digitale Bilddaten im Sichtfeld und/oder eine mit der Umgebung mitgeführte Darstellung in das Sichtfeld einzuspielen und/oder virtuelle Inhalte als Bilddaten auf den Monitor (50) zu leiten.

6.  Monitorsystem nach einem der vorhergehenden Ansprüche, **dadurch gekennzeichnet, dass** der erste Übertragungskanal (52) von der ersten Übertragungseinheit (42) zu dem Datenempfänger (48) und/oder dem Leistungsempfänger (46) ausgebildet ist, und/oder dass ein zweiter Übertragungskanal (54) von einer zweiten Übertragungseinheit (44) zu dem Datenempfänger (48) und/oder dem Leistungsempfänger (46) ausgebildet ist.

7.  Monitorsystem nach einem der vorhergehenden Ansprüche, **dadurch gekennzeichnet, dass** der erste Übertragungskanal (52) als ein optischer Übertragungskanal, insbesondere im IR-Bereich, ausgebildet ist.

8.  Monitorsystem nach einem der Ansprüche 3 bis 6, **dadurch gekennzeichnet, dass** der zweite Übertragungskanal (54) als ein optischer Übertragungskanal, insbesondere im IR-Bereich, ausgebildet ist, insbesondere wobei die zweite Übertragungseinheit (44) zur Umwandlung von elektrischer Leistung in optische Leistung und der Leistungsempfänger (46) zur Umwandlung von optischer Leistung in elektrische Leistung ausgebildet ist.

9.  Monitorsystem nach einem der vorhergehenden Ansprüche, **dadurch gekennzeichnet, dass** der erste Übertragungskanal (52) für eine serielle Übertragung von Bilddaten, insbesondere von komprimierten Bilddaten, ausgebildet ist.

10. Monitorsystem nach einem der vorhergehenden Ansprüche, **dadurch gekennzeichnet, dass** eine Bewegung des Auges (10) mit einem System zur Verfolgung von Augenbewegungen verfolgbar ist und/oder dass der Monitor (50) mit Beschleunigungssensoren ausgebildet ist, welche mit der Kamera (40) und/oder der ersten Übertragungseinheit (42) so zusammenwirken, dass eine Bildstabilisierung erfolgt.

11. Monitorsystem nach einem der vorhergehenden Ansprüche, **dadurch gekennzeichnet, dass** ein Kompressionsalgorithmus für die Bilddaten an eine typische Schärfenverteilung des Auges (10) angepasst ist.

**12.** Monitorsystem nach Anspruch 11, **dadurch gekennzeichnet, dass** der Kompressionsalgorithmus so an die typische Schärfenverteilung des Auges angepasst ist, dass eine Weitergabe von unnötig detailreichen Bildinformationen an das äußere Gesichtsfeld zumindest reduziert ist.

**13.** Monitorsystem nach einem der vorhergehenden Ansprüche, **dadurch gekennzeichnet, dass** auf dem Monitor (50) dargestellte Bilddaten so verzerrt werden, dass Bildelemente in ausgewählte Bereiche der Netzhaut (26) projizierbar sind.

**14.** Monitorsystem nach einem der vorhergehenden Ansprüche, **dadurch gekennzeichnet, dass** der Leistungsempfänger (46) und der Datenempfänger (48) in einer Einheit integriert, insbesondere integriert in den Monitor (50) ausgebildet sind.

**15.** Monitorsystem nach einem der vorhergehenden Ansprüche, **dadurch gekennzeichnet, dass** die erste Übertragungseinheit (42) und die zweite Übertragungseinheit (44) in einer Einheit integriert, insbesondere integriert in die Kamera (40) ausgebildet sind.

**16.** Monitorsystem nach einem der vorhergehenden Ansprüche, **dadurch gekennzeichnet, dass** die Kamera (40), insbesondere ein Scharfstellen der Kamera (40) über einen Ziliarkörper (34) des Auges (10) ansteuerbar ist und/oder dass die Kamera (40), insbesondere ein Scharfstellen der Kamera (40) über ein System zur Verfolgung von Augenbewegungen ansteuerbar ist.

**Claims**

**1.** Monitor system (100) for a human or animal eye (10), at least comprising

a monitor (50) which can be implanted in the eye (10) and is connected to a power receiver (46) and a data receiver (48),
a camera (40) for recording image data, which is connected to a first transmission unit (42) for processing, in particular compressing, and for wirelessly transmitting at least the image data via a first transmission channel (52) to the data receiver (48),
wherein the data receiver (48) is configured for receiving the image data and forwarding it to the monitor (50),

wherein the monitor (50) is configured to display image data recorded by the camera (40),
wherein the monitor (50) is designed to be arranged in place of the lens (22) in the lens sac (36) of the eye (10), in particular wherein the power receiver (46) and/or the data receiver (48) is arranged on a side of the monitor (50) facing the outside world, and wherein a projection apparatus is provided on a side of the monitor (50) facing an inside of the eye (10), whereby the image data can be projected onto the retina (26), **characterized in that**
the monitor (50) has an opening (56) with the form and action of a pupil, whereby light from outside the eye (10) falls onto the retina (26).

**2.** Monitor system according to claim 1, **characterized in that** the power receiver (46) is configured to receive electromagnetic signals, in particular optical signals or radio signals, and to convert the electromagnetic signals into electric power for the data receiver (48) and the monitor (50).

**3.** Monitor system according to claim 1 or 2, **characterized in that** a second transmission unit (44) is provided for the wireless transmission of power for the electrical supply of the data receiver (48) and/or the monitor (50) via a second transmission channel (54) to the power receiver (46).

**4.** Monitor system according to any one of the preceding claims, **characterized in that** the camera (40) is arranged outside the eye (10).

**5.** Monitor system according to any one of the preceding claims, **characterized in that** the data receiver (48) is configured to bring digital image data in the field of vision and/or a representation brought by the environment into the field of vision and/or to feed virtual content as image data to the monitor (50).

**6.** Monitor system according to any one of the preceding claims, **characterized in that** the first transmission channel (52) is formed from the first transmission unit (42) to the data receiver (48) and/or the power receiver (46), and/or **in that** a second transmission channel (54) is formed from a second transmission unit (44) to the data receiver (48) and/or the power receiver (46).

**7.** Monitor system according to any one of the preceding claims, **characterized in that** the first transmission channel (52) is configured as an optical transmission channel, in particular in the IR range.

**8.** Monitor system according to any one of claims 3 to 6, **characterized in that** the second transmission channel (54) is configured as an optical transmission channel, in particular in the IR range, in particular wherein the second transmission unit (44) is configured to convert electric power into optical power and the power receiver (46) is configured to convert optical power into electric power.

9. Monitor system according to any one of the preceding claims, **characterized in that** the first transmission channel (52) is configured for a serial transmission of image data, in particular compressed image data.

10. Monitor system according to any one of the preceding claims, **characterized in that** a movement of the eye (10) can be tracked by a system for tracking eye movements and/or **in that** the monitor (50) is configured with acceleration sensors which interact with the camera (40) and/or the first transmission unit (42) so that an image is stabilized.

11. Monitor system according to any one of the preceding claims, **characterized in that** a compression algorithm for the image data is adapted to a typical sharpness distribution of the eye (10).

12. Monitor system according to claim 11, **characterized in that** the compression algorithm is adapted to the typical sharpness distribution of the eye in such a way that a transmission of unnecessarily detailed image information to the outer field of vision is at least reduced.

13. Monitor system according to any one of the preceding claims, **characterized in that** image data displayed on the monitor (50) is distorted so that image elements can be projected into selected areas of the retina (26).

14. Monitor system according to any one of the preceding claims, **characterized in that** the power receiver (46) and the data receiver (48) are integrated into one unit, in particular integrated into the monitor (50).

15. Monitor system according to any one of the preceding claims, **characterized in that** the first transmission unit (42) and the second transmission unit (44) are integrated into one unit, in particular integrated into the camera (40).

16. Monitor system according to any one of the preceding claims, **characterized in that** the camera (40), in particular a focusing of the camera (40), can be controlled by a ciliary body (34) of the eye (10), and/or **in that** the camera (40), in particular a focusing of the camera (40), can be controlled by a system for tracking eye movements.

**Revendications**

1. Système de dispositif de surveillance (100) pour un oeil humain ou animal (10), comprenant au moins

   un dispositif de surveillance (50) implantable dans l'œil (10) qui est connecté à un récepteur de puissance (46) et à un récepteur de données (48),
   une caméra (40) pour l'enregistrement de données d'image qui est connectée à une première unité de transmission (42) pour la préparation, en particulier la compression, et pour la transmission sans fil d'au moins les données d'image au récepteur de données (48) par l'intermédiaire d'un premier canal de transmission (52),
   dans lequel le récepteur de données (48) est conçu pour recevoir les données d'image ainsi que pour les transmettre au dispositif de surveillance (50),
   dans lequel le dispositif de surveillance (50) est conçu pour représenter des données d'image enregistrées avec la caméra (40),
   dans lequel le dispositif de surveillance (50) est prévu pour pouvoir être disposé dans le sac capsulaire (36) de l'œil (10) à la place de la lentille (22), en particulier dans lequel le récepteur de puissance (46) et/ou le récepteur de données (48) est disposé sur un côté du dispositif de surveillance (50) qui est tourné vers le monde extérieur, et dans lequel un appareil de projection est prévu sur un côté du dispositif de surveillance (50) qui est tourné vers un côté intérieur de l'œil (10), moyennant quoi les données d'image peuvent être projetées sur la rétine (26),
   **caractérisé en ce que**
   le dispositif de surveillance (50) présente une ouverture (56) ayant la forme et l'effet d'une pupille, moyennant quoi de la lumière tombe d'un côté extérieur de l'œil (10) sur la rétine (26).

2. Système de dispositif de surveillance selon la revendication 1, **caractérisé en ce que** le récepteur de puissance (46) est conçu pour recevoir des signaux électromagnétiques, en particulier des signaux optiques ou des signaux radio, et pour convertir les signaux électromagnétiques en puissance d'alimentation électrique pour le récepteur de données (48) et le dispositif de surveillance (50).

3. Système de dispositif de surveillance selon la revendication 1 ou 2, **caractérisé en ce qu'**une seconde unité de transmission (44) est prévue pour la transmission sans fil de puissance d'alimentation pour l'alimentation électrique du récepteur de données (48) et/ou du dispositif de surveillance (50) via un second canal de transmission (54) au récepteur de puissance (46).

**4.** Système de dispositif de surveillance selon l'une quelconque des revendications précédentes, **caractérisé en ce que** la caméra (40) est disposée à l'extérieur de l'œil (10).

**5.** Système de dispositif de surveillance selon l'une quelconque des revendications précédentes, **caractérisé en ce que** le récepteur de données (48) est conçu pour introduire des données d'image numériques dans le champ de vision et/ou une représentation apportée par l'environnement dans le champ de vision et/ou pour diriger des contenus virtuels en tant que données d'image sur le dispositif de surveillance (50).

**6.** Système de dispositif de surveillance selon l'une quelconque des revendications précédentes, **caractérisé en ce que** le premier canal de transmission (52) est formé par la première unité de transmission (42) vers le récepteur de données (48) et/ou le récepteur de puissance (46), et/ou **en ce qu'**un second canal de transmission (54) est formé par une seconde unité de transmission (44) vers le récepteur de données (48) et/ou le récepteur de puissance (46).

**7.** Système de dispositif de surveillance selon l'une quelconque des revendications précédentes, **caractérisé en ce que** le premier canal de transmission (52) est conçu en tant que canal de transmission optique, en particulier dans le domaine IR.

**8.** Système de dispositif de surveillance selon l'une quelconque des revendications 3 à 6, **caractérisé en ce que** le second canal de transmission (54) est conçu en tant que canal de transmission optique, en particulier dans le domaine IR, en particulier dans lequel la seconde unité de transmission (44) est conçue pour convertir de la puissance électrique en puissance optique et le récepteur de puissance (46) est conçu pour convertir de la puissance optique en puissance électrique.

**9.** Système de dispositif de surveillance selon l'une quelconque des revendications précédentes, **caractérisé en ce que** le premier canal de transmission (52) est conçu pour une transmission en série de données d'image, en particulier de données d'image comprimées.

**10.** Système de dispositif de surveillance selon l'une quelconque des revendications précédentes, **caractérisé en ce qu'**un mouvement de l'œil (10) peut être suivi par un système de suivi des mouvements de l'œil et/ou **en ce que** le dispositif de surveillance (50) est conçu avec des capteurs d'accélération qui coopèrent avec la caméra (40) et/ou la première unité de transmission (42) de sorte qu'une stabilisation d'image soit effectuée.

**11.** Système de dispositif de surveillance selon l'une quelconque des revendications précédentes, **caractérisé en ce qu'**un algorithme de compression pour les données d'image est adapté à une distribution de netteté typique de l'œil (10).

**12.** Système de dispositif de surveillance selon la revendication 11, **caractérisé en ce que** l'algorithme de compression est adapté à la distribution de netteté typique de l'œil de sorte qu'une transmission d'informations d'image inutilement riches en détails au champ visuel extérieur soit au moins réduite.

**13.** Système de dispositif de surveillance selon l'une quelconque des revendications précédentes, **caractérisé en ce que** des données d'image représentées sur le dispositif de surveillance (50) sont déformées de sorte que des éléments d'image puissent être projetés dans des zones sélectionnées de la rétine (26).

**14.** Système de dispositif de surveillance selon l'une quelconque des revendications précédentes, **caractérisé en ce que** le récepteur de puissance (46) et le récepteur de données (48) sont intégrés dans une unité, en particulier intégrés dans le dispositif de surveillance (50).

**15.** Système de dispositif de surveillance selon l'une quelconque des revendications précédentes, **caractérisé en ce que** la première unité de transmission (42) et la seconde unité de transmission (44) sont formées dans une unité intégrée, en particulier intégrée dans la caméra (40).

**16.** Système de dispositif de surveillance selon l'une quelconque des revendications précédentes, **caractérisé en ce que** la caméra (40), en particulier une mise au point de la caméra (40), peut être commandée par un corps ciliaire (34) de l'œil (10) et/ou **en ce que** la caméra (40), en particulier une mise au point de la caméra (40), peut être commandée par un système de suivi des mouvements de l'œil.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102005032989 A1
- US 2018071146 A1
- WO 2015081298 A1